# EUROPEAN PATENT APPLICATION

(11) **EP 2 317 311 A2**
(43) Date of publication of application: **04.05.2011**
(21) Application number: 10275094.0
(22) Date of filing: 13.09.2010
(51) Int. Cl.: G01N 33/00, G08B 21/14, G08B 25/00

(54) **Sensing network and method**

(30) Priority: 11.09.2009 GB 0916000
(71) Applicant: SELEX Galileo Ltd, Basildon Essex SS14 3EL (GB)
(72) Inventor: Lepley, Jason, Basildon, Essex SS14 3EL (GB); Bourdon, Graham, Basildon, Essex SS14 3EL (GB); Friedrich, Douglas, Basildon, Essex SS14 3EL (GB); Rudd Alison C Dr., Reading , Berkshire RG6 6BB (GB); Belcher Stephen Prof., Reading , Berkshire RG6 6BB (GB); Robbins Alan G. Prof., Guildford, Surrey GU2 7HX (GB)
(74) Representative: Wojcik, Lucy Eleanor

(57) **Abstract**

A system and method are described for detecting toxic gas releases in an urban environment. The system comprises a series of repositionable remote sensor nodes that are located in an environment containing a toxic gas said sensor nodes including a chemical sensor and/or a meteorological sensor. The sensor nodes gather data relating to the type of gas released and the surrounding atmospheric conditions and relay the information back to a data processing hub where the data is collated and analysed to produce an indication of the likely spread of the gas plume, the likely danger to the surrounding population and an indication of the release point of the gas.

## Description

The invention relates to a sensing network and method. More specifically, but not exclusively, it relates to a dynamic deployment system for planning, monitoring and sourcing the location of toxic gas leaks using an ad-hoc sensor network operating at the urban city street level (or meteorological microscale).

The ability to analyse the chemical composition or quality of air samples in a controlled environment is easily demonstrated, but the problem becomes extremely complex when translated to an unconstrained outdoor environment. Here the sensors are faced with the need to identify very low particle counts, often in the presence of high levels of benign pollutants and rapidly changing turbulent meteorological conditions.

Sensing systems for detecting and predicting the location and direction of travel of gas clouds or plumes are known on a macro scale. There are known systems for example, such as the Meteorological Office plume prediction reports (CHEMET), based on handheld point chemical sensors. However, these CHEMET agency prediction systems operate on a 'down-field' aspect of a toxic contamination event on the meteorological mesoscale (5 to ∼200 km). It is a disadvantage of this system that it does not provide either views to the short range urban city street-level dispersion of the toxic plume or the ability to reverse predict so as to determine the location of the toxic release given a mid-field event marked by a concentration of casualties.

According to the invention there is provided a system for locating the position of a gas release comprising a series of remote sensors positioned in known locations and data processing means, the remote sensors being capable of detecting the gas released and transmitting data relating to the gas to the data processing means, the data processing means receiving input data and processing said data according to known meteorological data so as to indicate the location of the gas release.

Preferably the meteorological data relates to urban or small scale environments such as city centre locations.

Preferably, the sensors are capable of detecting physical attributes of the gas release such as concentration and detecting the actual gas released.

According to the invention there is further provided a method for estimating and monitoring the dispersion of an airborne contaminant plume and locating the source of the contaminant comprising the steps of: locating a series of remote sensors at known locations within a given area, the sensors being capable of detecting and quantifying the amount of gas airborne, monitoring the output of the sensors using appropriate monitoring means, applying meteorological data to the information out put by the sensors and triangulating a predicted position of the gas release.

In this way, the system and method overcomes the disadvantages of hereto know systems such as those described above.

The invention will now be described with reference to the accompanying diagrammatic drawings in which:
Figure 1 is an overview of one form of the invention 1, showing remote sensor nodes 2 wirelessly communicating with a gateway 3, said gateway transmitting the received signals to a data processing hub 4 for onward processing and production of a prediction, each node comprising a meteorological sensor 5 and a chemical detecting sensor 6;
Figure 2 is a block diagram showing a breakdown of the data processing hub 3 of Figure 1; and
Figure 3 is a block diagram showing the use of sensor nodes 2 for data gathering and a gateway node 4 for data processing, the gateway node 4 calculating plume position, threat to population etc.

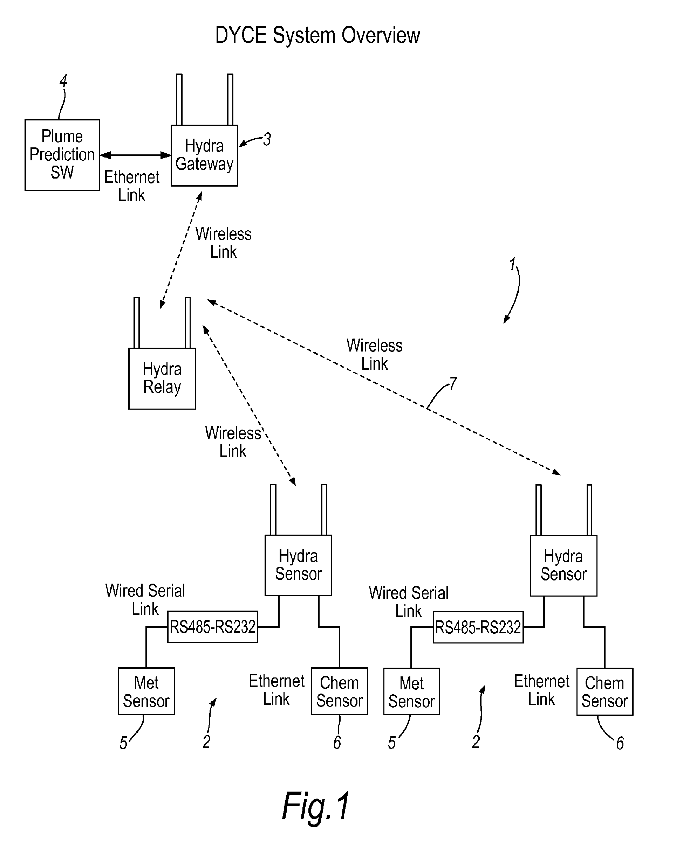

As shown in Figure 1, the system comprises two or more sensor nodes 2. Each sensor node 2 having: a means 7 of communicating data or information with other nodes; a means 6 of detecting the airborne contaminant and of measuring the concentration of the airborne contaminant; a means 5 of measuring the speed and direction of the wind; a means of determining its' location such as GPS. Figure 1 depicts the sensor nodes 2 and how they interconnect. It is expected that the data is communicated wirelessly between the sensor nodes 2 and to the gateway node 4 although it will be appreciated that wired or other communications methods may also be suitable.

Following the suspected release of an airborne contaminant the sensor nodes 2 are deployed in and around the environment and will gather data on the localised nature and concentration of the contaminant and of the meteorological conditions local to that sensor node 2. This data will be communicated across the network to a gateway node 4. The data may be communicated directly to the gateway node 4 in what is known as a 'star' network configuration. In another configuration the data may be communicated indirectly to the gateway node in what is known as a multi-hop network. The nature of the communications may be dictated by the communications reach of the sensor nodes or the presence of buildings or other clutter that limits the communications reach of the sensor nodes. To assist in the formation of a network that can communicate the data across the network in a manner that befits the geographical spread or level of clutter within the environment under consideration it may be necessary to employ the use of communications relay nodes.

A gateway sensor node 4 will have a means of connecting the network to a computer that can process the data from the sensors to:
- Assess the current state of the contaminant plume
- Extrapolate the future motion of the contaminant plume
- Back-calculate the location of the contaminant source or sources

The gateway 4 or one of the sensor nodes 2 or a computer connected to one of the sensor nodes 2 may also have a means of presenting this information to a user.

In another implementation the means of processing the data may be resident on one of the sensor nodes 2 within the network.

In another implementation the means of processing the data may be distributed across two or more of the sensor nodes 2 within the network.

In addition to the sensor data the plume prediction algorithms may also need information relating to the topography of the environment under consideration. This information may be extracted from a database or estimated.

The sensor nodes 2 may collectively have a means of evaluating whether they are appropriately placed and / or of computing a more appropriate location for them to be placed given knowledge of the current or evolving state of the contaminant plume and of the meteorological conditions. This process is referred to in the summary as deployment planning.

In the initial implementation the sensor nodes 2 are expected to be manually deployed. In another implementation the sensor nodes 2 may contain a means of mobilising themselves either through manual piloting or autonomously based on the outputs of the deployment planning algorithm.

The system 1 employs a small network of ad-hoc deployable sensor nodes 2 that are able to monitor and react to changing local conditions and chemical data content, such that end-users may dynamically optimise their locations.

In one embodiment of the invention described it is assumed that the airborne contaminant is a toxic gas or a hazardous chemical. However, it will be appreciated that the system and method described are equally applicable to detection and prediction of plume direction of any airborne contaminant capable of detection by suitable sensors 2.

In the embodiment described, it is necessary to first understand the nature of the problem and how the response to incidents will be addressed by emergency services. This understanding will feed into a specific set of user requirements for a rapidly deployable sensing solution.

The system described will Detect-to-Warn - that is, detect levels of chemicals at lower than Immediately Dangerous to Life or Health (IDLH) and warn the system operators such that safety precautions can be taken (evacuation or donning of CBRN personnel protective equipment).

From the deployment perspective chemical sensors 6 (which will assume to describe both chemical detection and chemical monitoring systems) have generally been broken down into 2 classes:
1. Stand-off - Sensors (e.g. LIDAR) able to provide a capability at a distance from the chemical hazard/plume zone.
2. Point-of-Contact - Sensors that must be in direct contact with the chemical hazard/plume zone. An integrated network of multiple sensors is required to give wide area coverage.

Stand-off sensors (SoSs), most of which are spectroscopic based (e.g. IR, THz, Raman, *etc*.) have traditionally been the choice systems when considering remote deployment because they can be robotically programmed to provide wide area coverage and in some cases can be operated in a "safe zone" away from the hazard. These sensors are not optimal for the detection and tracking of plumes at street level in an urban environment.

Point-of-Contact sensors (POCSs), by their nature, have had limited remote deployment capability since they must be man or vehicle borne in order to make contact with the hazard/plume zone. Effective wide area coverage can only be provided by deployment in large numbers. In this respect, the combination of logistical support requirements with the Size, Weight, Power & Cost (SWPC) *versus* performance trade-off's have proven a handicap for POCSs. The vision for effective wide area remote deployment is the networking of static unattended POCSs. This can be facilitated by a sensor design which meets a SWPC *versus* performance trade-off specification, to be deployed in sufficient density for wide area coverage. Effectively one creates a "stand-off" capability by the sheer number density of sensors in the network.

One form of sensor that may be employed uses Field-Asymmetric Ion Mobility Spectrometry (FAIMS) technology. In this way chemical sensor networks may be applied in applications ranging from homeland security and defense through to occupational hygiene and environmental monitoring. Given the ongoing advances in communication network technology and the drive in the development of unattended systems containing suites of sensors (motion, seismic, imaging, metrological, etc.).

POCSs may be broadband, detecting a wide range of threat agents, or for applications where a narrow range of threat agents are to be detected (e.g. chemical factory gas leaks where the escaping gas will always be the same chemical) a highly specific narrowband sensor can be used. The narrow band sensors will be more sensitive than the broadband sensor over the narrow sensing band.

In one embodiment of the invention, the system allows for identification of the location of the source and the location of regions which are hazardous.

In some instances the location of the source may be obvious, as there will be visible signs of either the plume itself or other indicators of the source of the plume, such as a container.

In other cases, and particularly for malicious releases where the instigator may attempt to hide or obscure the source, the approximate location of the source may only become evident from secondary signs (e.g. location of casualties). In this instance, the responders need additional information to locate the source. With a dense extant network of chemical and met sensors it may be possible to back-track the data to estimate the sources location. In the absence of such a sensor network (which is more likely at present), the responders can only rely on the wide scale met data (if this can be made available in a timely manner), spot chemical measurements and visible signs of the release.

Particularly within complex urban environments, the turbulence of wind flows within the 'urban canyon' can take many forms, ranging from efficient mixing of the agent with the atmosphere to movement of concentrated pockets of agents. In these cases it has been shown that attempting to localise the source using wide scale met data can produce variable or inaccurate results. A key requirement in locating the hazard is therefore an understanding of the local environment and the way in which wind will be channeled along streets and between buildings. This understanding is also essential in deriving an accurate prediction of the plume dispersion, as will be discussed later.

In addition to locating a single source, response agencies may also look at whether similar episodes are being reported elsewhere and take action to ensure that information is disseminated as appropriate.

In one embodiment of the invention, the system identifies the chemicals contained within the plume.

Identifying the chemical plume is critical both for the real-time response efforts and for input into the model nowcasting and forecasting. Assuming the approximate location of the release is known, responders need to make an accurate assessment of the identity of the release so as to ascertain the nature of the threat involved and its likely risk to humans.

To aid the immediate response of a chemical release, the chemical detector must be capable of:
- Accurately detecting and classifying a broad range of species of interest.
- Being small, having low power consumption and being capable of mobile deployment.
- Rapid detection and identification of the agent.

Chemical sensors typically fall into two categories. The first is the large and cumbersome lab-based analysis techniques such as mass spectrometry or ion mobility spectrometry. These typically provide a very accurate analysis of a chemical sample; however they tend to be slow and only practical for use in specialised laboratories. The second category includes some new technologies that are optimised to detect specific groups of chemicals, rapidly and at low concentrations.

FAIMS sensors provide a capability more in line with that achievable in laboratory equipment, whilst being field deployable. With suitable training the FAIMS sensor can classify and detect a wide range of chemical species, and achieve this with a very low FAR.

Detection in a new environment will be hampered by the presence of other contaminants. For example, within an urban environment there may be considerable levels of hydrocarbons and diesel particulates in the atmosphere and detecting an unknown species with such high levels of background clutter will be difficult.

In one embodiment of the invention, the system predicts how the agent will be dispersed, providing the operators with a prediction of the plume concentration at different locations within the environment.

Once released, the agent will disperse into the local environment and then be carried by wind flow into the wider environment. The wind speed and direction will be governed both by the wider scale wind conditions and also by the topography of the local environment. Local flows can carry the plume in unexpected directions; for example, in some conditions a plume can stagnate or suddenly change direction. Flows can also vary dramatically depending on the height above ground.

In attempting to model and predict the direction of the plume in such environments it is necessary to have sufficient data from which to make the assessment. During the immediate response phase to the incident, it is necessary to gather as much data from the environment as possible if we are to make accurate predictions on the dispersion of the plume. Placing multiple sensors within the area which are capable of measuring meteorological conditions will assist in providing this data; however there are several requirements that fall out of this, namely:
- Plume dispersion cannot be accurately predicted from a single sensor; and as such multiple sensors collaborate to provide a prediction.
- The location of each of the sensors is established with some accuracy. Preferably this will be correlated with maps or topographical information on the environment.
- The sensors will be deployed by first responders, namely users who will have been trained in their deployment. However the responders will need to make a rapid decision on the placement of each sensor and consequently this placement may be non-optimal. The sensor network will therefore be able to feedback to the user when they are inappropriately placed.
- Information from extant sensors may be used to augment the prediction model.
- Key meteorological data of interest will be wind speed and direction at multiple sensor locations. Additional information that might be of use includes temperature, humidity and barometric pressure.

In one form of the invention, the system provides an estimate of the risk to the population.

The information obtained by the system to address the requirements above will provide an estimate as to the risk the chemical release poses to the population, in terms of:
- Toxicological evaluation of the agent involved.
- Estimate on plume dispersion and the likely lethality or threat to health of the dispersing plume.
- Deposition and long term effects of the agents.
- Necessity of an evacuation, and likely sectors to evacuate and routes to take based on the known evolution of the plume.

In summary of the above sections, one form of the invention aims to fulfil the following roles:
- To provide real-time local information to emergency services during the critical immediate response phase to a chemical release, providing a detailed localised picture of the plumes location, dispersal and effects.
- Identify the chemical agents involved and measure the concentrations at the sensor node locations. This will assist in understanding the danger level of the hazard.
   ○ For use in the complex outdoor environments envisaged in this project, the sensor would need to identify the hazardous agents, which could be present in transient concentration levels, in the presence of background clutter.
- Measure the meteorological data at the sensor node locations and use this to:
   ○ Model the dispersion characteristics of the plume flow.
   ○ Predict the fate of the plume.
   ○ Inverse model the location and strength of the hazard source.
- Determine when nodes are positioned at suboptimal locations and provide recommendations for where nodes should be deployed.
- Estimate the risk the chemical release poses to the population, identifying the likely lethality, threat to health and necessity of evacuation.

In use the system will be deployed as follows:
1. Responders turn up at the scene of a chemical release incident (determined by the location of casualties or other means): the location, nature and dispersion of the chemical agent are initially unknown.
2. The responders deploy sensor nodes 2 at locations in and around the area where the effects of the chemical release are observed
3. The nodes 2 each measure the meteorological conditions and (as yet unknown) chemicals present at their locations.
4. The sensor nodes 2 communicate their chemical and met data along with their locations to a gateway node 4 which uses this information to provide, to the user, information on:
   a. Chemical agents detected (based on an assessment against a known list of chemical warfare agents (CWAs), toxic industrial chemicals (TICs), toxic organic compounds (TOCs), etc.).
   b. An estimate of the current plume condition (area of coverage, concentration, direction of movement, etc.).
   c. Forecast the fate of the plume to predict how it will evolve.
   d. Inverse model the evolution of the plume to localise the source.
   e. How well the network is achieving the mission objectives (as determined by 4.a to 4.d above) and advise on relocating some or all of the nodes if necessary.
5. The data above can be compared with wider scale met predictions of the plume's fate.

One of the key operational features envisaged for the invention is to optimise its use by a limited set of sensor nodes 2 by ensuring that they are each located in a position that is providing useful data. This might, for example, be used to constrain the minimum and maximum separation of any two nodes, to reduce redundancy of effort and minimise likelihood of lost communications links respectively. It will also ensure that nodes that are providing little or no useful information are re-deployed to a more viable location. The situation of a node receiving little information of use might result from it being inappropriately placed for several reasons:
- Proximity to an object that is shielding the node 2 from air flow.
- Proximity to an exhaust vent, humid air flow or other source of extreme background clutter that will impair the sensor's 2 ability to detect chemicals.
- The sensor node 2 is appropriately placed and receiving low levels of background clutter yet is not detecting the chemical agent being picked up by other sensing nodes 2. This node 2 may be upwind of the plume flow and hence may never detect the plume or it may be downwind but too far from the plume. Redeployment of the node might enhance the information derived from the network.

This information will be assessed both by the plume dispersion prediction means or algorithms and by the mission and redeployment planning software means.

Thus far, there has been an implication that the sensors remain stationary until moved by the responders. This is, at least initially, the anticipated mode of operation. In a further form of the invention the sensors are placed on mobile platforms such as vehicles or UGVs (unmanned ground vehicles); the latter may even be guided autonomously by the mission planning software.

A block diagram of one form of the invention platform is depicted in Figure 1.

The platform 1 comprises a number of sensor nodes 2, each of which houses three main hardware components: a CISP sensor platform, a chemical sensor 6 and a meteorological sensor 5.

As shown in Figure 1, a high performance CISP (Common Integrated Sensors Processor) is used. The CISP comprises a high performance processor with multiple networking options (which may include 802.11 a/b/g, Ethernet and GPRS) and multiple 'personality' cards that enable a wide range of sensors to be attached, including a Global Positioning System (GPS) sensor. CISP has been designed to operate under the extreme conditions of a military environment and as such is integrated into a sensor node product known as HYDRA.

CISP's ability to operate with different networking protocols are highly beneficial to the complex urban environments envisaged.

A sensor node 2 capable of stand alone chemical sensing and containing a FAIMS chemical sensor may be used. It is uniquely capable of detecting and identifying the full spectrum of chemical warfare agents, TICs and TOCs in a mobile, field deployable platform. Other suitable sensors may be used.

A compact meteorological sensor 5 is used in tandem with the chemical sensing sensor 6. The sensor 6 incorporates sensors to measure wind speed and direction, temperature, humidity and barometric pressure.

A gateway 3 is provided as a direct communications link between the CISP network and the user and comprises a node and a user interface device.

The data processing means or algorithms ported onto the CISP platform fall into three categories:
- Hazard Assessment - Takes data from the chemical sensor and feeds this to the HMI to provide the user with knowledge of the chemical agent(s) detected.
- Plume Prediction - Combines data from the multiple Met and chemical sensors to predict and inverse model the flow of the plume.
- Mission/Deployment Planning - Compares information obtained from the sensors with the mission expectations and makes a decision (based on knowledge of the plume) on redeployment.

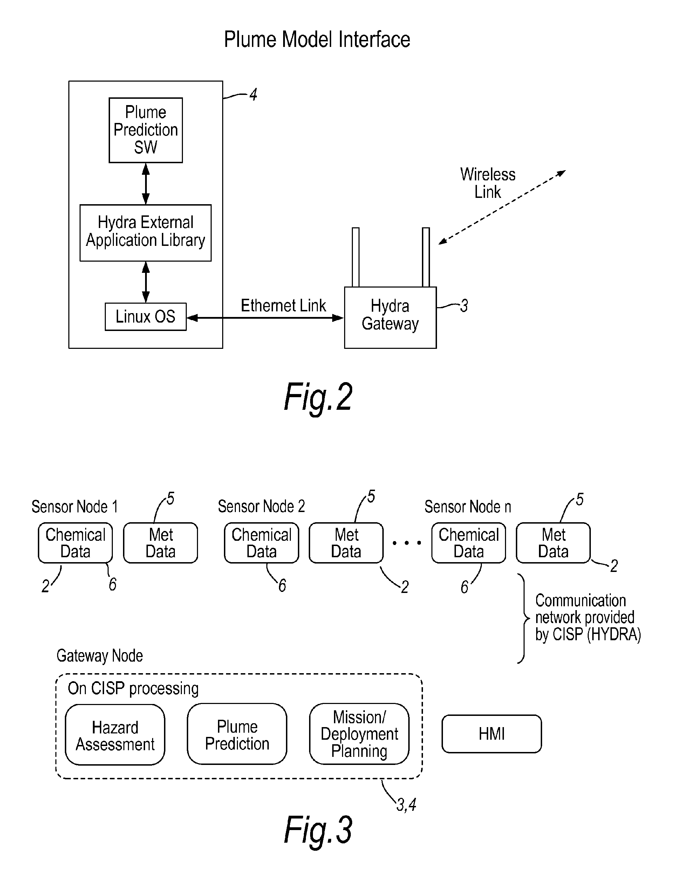

A block diagram of this implementation is shown in Figure 3. The current preferred implementation is to use the sensor nodes for the purpose of data gathering and to communicate this data to a gateway node. The gateway node provides the prime interface with the user and will act as the primary processing node on which the algorithms will run.

The system as described above is designed to use a number of different forward dispersion models to accommodate the range of applications that might be encountered in practice; i.e. from dense, urban conditions to open rural terrain, although it is the former that is the main focus of interest. Variants of the models can also be selected according to the level of information available in any particular application. Inverse modelling is based on the chosen forward model and uses statistical methods to adjust source conditions (i.e. location and source strength) to provide an optimum correspondence between the observations and the corresponding predictions. This process also estimates the uncertainty associated with the optimised source conditions.

### Forward models

The default model or the system may be an urban dispersion model using the street network approach. This applies a mass balance to the flow through each street segment and intersection comprising the network, based on the inflows and outflows in the streets and the exchanges with the atmosphere above roof level. Empirical relationships are used to relate these flows to the local urban topography (i.e. building shapes, sizes, etc.) and the meteorological conditions above the urban 'canopy'. Minimum input requirements for the latter are the mean wind speed and direction at a single location clear of any adverse influences from surrounding buildings, though more comprehensive information is preferred to provide a more precise representation of the flow above roof level. Conversion of the urban topography in a region of interest, which might well measure 1 x 1 km, into the form used by the network model is complex and would normally be carried out off-line, with the resulting network model stored in a data-base. If such a pre-processed model does not exist for a particular application and cannot be generated in the time available (which will often be the case in real time) then the next option may be to use a generic network model based on a regular array of cubes. The height of the cubes is made equal to the mean height of the buildings in the region of interest and the array designed to cover the same proportion of surface area as the actual site buildings.

If use of the network model is judged to be unsatisfactory or the relevant data for its use is not to hand then an alternative Gaussian plume model may be used. This would be the model of choice for application to open terrain. The Gaussian model is provided with a number of pre-set application choices, these being standard plume spread relationships for rural and urban terrain, plus an additional option for dense urban conditions. Input for the latter includes the mean building height and the proportion of the surface area covered by buildings, though default values will be provided for these parameters that can be used when such information is not available. The default values will be for typical UK conditions, though they can be reset to other situations as need be.

### Inverse modelling

The inverse modelling procedure is independent of the choice of forward model, though it does require information from it. Concentration data are provided at regular intervals from the detectors that have been deployed to monitor an incident, together with meteorological data. The objective of the inverse modelling is to position a source and specify its strength so that the forward model predicts the observations with minimum error. This is done in an iterative manner, starting from initial estimates that will generally be the output of the evaluation at the previous time step (i.e. when the previous set of observations was analysed). The measure of error that is minimised in order to estimate the source terms is the sum of the weighted mean square differences between the model predictions and the observations, referred to as a 'cost or penalty' function. The weighting describes the uncertainty in the differences between predictions and observations; it has three components: measurement error, sampling error and modelling error. The first will generally be well established for a particular measurement technique. The second reflects natural variability in the dispersion process and depends on the interval over which concentrations have been measured. In many cases, where the emission rate and meteorological conditions are relatively steady, it decreases as more data is collected. Modelling error quantifies how well the model performs when judged against error-free data (i.e. when the other sources of error are zero) and, in general, this will only be known in some overall, though probably application related context. The weightings not only control the influence of particular measurements on the inversion process but also determine the uncertainty attached to the final predictions of source strength and position.

The ability of the inversion process to converge onto an optimal source strength and location depends on the quality of the data and part of the system methodology is to use an assessment of data quality to decide if any particular detector should be repositioned. Two useful data quality measures are the signal to noise ratio and the concentration fluctuation intensity (the ratio of the standard deviation to the mean value). A monitor can be judged to be effectively sited if the signal to noise ratio of the data stream it returns lies above a pre-defined threshold and the concentration fluctuation intensity below a second such threshold. Ineffectively sited monitors are eligible for repositioning, particularly if they fail the quality test on two successive occasions (assuming that one failure might occur due to the stochastic nature of the dispersion process). The output from the forward model provides the information needed to recommend areas in which it would be beneficial to add monitors, based on local sparseness of the sampling network, particularly in areas where concentration levels are predicted to be hazardous.

Poor data quality not only results in large uncertainty in source estimates but may cause the inversion algorithm to fail altogether. This is most likely to occur during the initial stages of an event (assuming a reasonably designed monitoring network), particularly if the time interval for acquiring data is short (in practice, below a few minutes). In such cases no source prediction is output and the incoming data is stored to be combined with the following data-set, effectively providing an average over twice the basic data time interval. This process will be continued until convergence is achieved. The number of time steps required then defines a new time interval that will be used throughout the remainder of the event. In this way, an acceptable time interval is determined by iteration, taking account of the nature of the event in progress.

The incoming data stream is treated in two ways as an event continues. Firstly, the operational sampling time interval is determined as described above and then each such interval is treated separately, yielding a set of source terms that is added to an array of all such predictions up to the current time. Secondly, the incoming data is used to update the rolling average of the reference wind conditions and the concentrations from each detector, and these averages are then used with the inversion algorithm. Again, the array of all such predictions is saved. The second method may require lateral dispersion parameters that are functions of the sampling time and additional lateral spread due to variations in sample mean wind direction is included for applications involving prolonged events.

If the source conditions are steady (or at most changing very slowly relative to the sampling interval) then the second procedure converges to the best estimate of the source conditions, with the uncertainty due to sampling time gradually decreasing to insignificance. In such cases, predictions from the first method tend to become scattered in an unbiased way about the converged values, the degree of scatter being consistent with the predicted uncertainty in the source estimates. A failure for the outputs to behave as described is taken to be an indication that the source terms or meteorological conditions are not steady and in such cases the output reverts to that from the first analysis procedure.

In this way, the invention described provides a method for estimating and monitoring the dispersion of an airborne contaminant plume and locating the source of the contaminant. The method incorporates planning and dynamically optimising in real-time the deployment of a sparsely populated ad-hoc sensor network within a built up (e.g. urban, city street) environment taking microscale (<2Km) meteorological data, topographical data of the street layout & buildings, sensor performance and RF environment data into consideration. This allows the 'first responder' emergency services to optimally deploy sensors around a mid-field event marked by a concentration of casualties thereby enabling the rapid identification and location the (unknown) contaminant source to be determined and to forecast the short range spread of the contaminant plume around the street-level environment.

## Claims

1. A system for locating the position of a gas release comprising a series of remote sensors positioned in known locations and data processing means, the remote sensors being capable of detecting the gas released and transmitting data relating to the gas to the data processing means, the data processing means receiving input data and processing said data according to meteorological data so as to indicate the location of the gas release.

2. A method for estimating and monitoring the dispersion of an airborne contaminant plume and locating the source of the contaminant comprising the steps of: locating a series of remote sensors at known locations within a given area, the sensors being capable of detecting and quantifying the amount of gas airborne, monitoring the output of the sensors using appropriate monitoring means, applying meteorological data to the information output by the sensors and triangulating a predicted position of the gas release.

3. A system or method according to claim 1 or 2 in which the sensors comprise a chemical sensor and a meteorological sensor.

4. A system or method according to any preceding claim in which the sensors are located remote from one another in an urban environment.

5. A system or method as hereinbefore described with reference to the accompanying diagrammatic drawings.
